# EUROPEAN PATENT APPLICATION

(11) **EP 3 050 488 A1**
(43) Date of publication of application: **03.08.2016**
(21) Application number: 14885968.9
(22) Date of filing: 19.09.2014
(51) Int. Cl.: A61B 1/00, G02B 23/24

(54) **ENDOSCOPE**

(30) Priority: 18.03.2014 JP 2014055376
(71) Applicant: Olympus Corporation, Hachioji-shi Tokyo 192-8507 (JP)
(72) Inventor: HIJIHARA, Kunihiko, Tokyo 192-8507 (JP); SAKAI Koji, Tokyo 192-8507 (JP)
(74) Representative: Gunzelmann, Rainer
(86) International application number: PCT/JP2014/074910
(87) International publication number: WO 2015/141039

(57) **Abstract**

An endoscope includes: an insertion portion including, on a distal end portion side, a bending portion that is bendable at least upward and downward; a cylindrical operation portion provided so as to be continuous with a proximal end side of the insertion portion, the operation portion having a first longitudinal axis substantially parallel to a longitudinal axis of the insertion portion and being capable of forming a part of a first grasping portion to be grasped by one hand of a user; a cylindrical extension portion provided so as to be continuous with a proximal end side of the operation portion, the extension portion having a second longitudinal axis extending in a downward bending direction of the bending portion and being capable of forming a part of the first grasping portion and a second grasping portion to be grasped by the one hand; and a bending lever including a first end and a second end, which has a first end portion pivotally provided at the operation portion, and a second end portion provided so as to extend from the first end portion in an upward bending direction of the bending portion, where a finger of the one hand grasping the first grasping portion or the second grasping portion is placed on the second end portion when operation to bend the bending portion is performed.

## Description

### Technical Field

The present invention relates to an endoscope including a bending lever for performing operation to bend a bending portion, at an operation portion.

### Background Art

Endoscopes that enable, e.g., observation to be performed by inserting an elongated insertion portion into a subject have been in wide use. Some of these endoscopes are of a type in which a bending portion is provided on the distal end side of the insertion portion. The bending portion is generally configured so as to perform bending operation by a bending operation apparatus provided at the operation portion being operated to pull/loosen a bending wire.

For example, Japanese Patent Application Laid-Open Publication No. 2006-110053 (hereinafter referred to as Literature 1) indicates an electronic endoscope in which a grip portion for grasping is provided at an operation portion joined to a proximal end of an insertion portion. At one side face on the rear end side relative to the grip portion of the operation portion, a dial portion, which is a first operation member, and an operation lever, which is a second operation member, are provided as bending operation means for operating the bending portion. The provision of the two types of operation members as the bending operation means enables quick bending operation to be performed by operating the operation lever with a thumb alone and also enables fine bending operation to be performed by pinching the dial portion from opposite sides and operating the dial portion.

Japanese Patent Application Laid-Open Publication No. 2007-190047 (hereinafter referred to as "Literature 2") indicates a body operation portion to be held by a surgeon with his/her hand to operate an endoscope. The body operation portion includes a casing, and the casing includes a body casing, a front casing and a rear casing. An insertion portion extends from the front casing, and a universal cord is drawn out from the rear casing.

A shaft end of a pulley drive shaft projects from each of opposite side portions of the body casing, an angle operation lever is provided at each of the shaft ends. Each angle operation lever extends in a direction perpendicular to the pulley drive shaft along a corresponding side face of the body casing. A distal end portion of each extending lever is flexed in a direction that is parallel to the pulley drive shaft, whereby a finger rest/operation portion to be operated with a finger of a hand is formed. It is described that when the front casing of the endoscope is grasped, the first finger is positioned on the finger rest/operation portion side, and when the rear casing is grasped, the thumb is positioned on the finger rest/operation portion side.

If a surgeon tries to insert the endoscope in Literature 1 or 2 to, for example, a nasal cavity to conduct an examination, generally, the insertion portion is inserted to the nasal cavity of the patient, for example, sitting on a chair, with the insertion portion kept substantially horizontal. Thus, the operation portion provided in alignment with a longitudinal axis direction of the insertion portion is grasped as illustrated in Figs. 12(A) and 12(B) in Literature 1, and an operation knob is operated with the thumb. In this grasping and operation state, the surgeon needs to keep the insertion portion horizontal by tilting the wrist forward in a posture in which the armpit is spaced from the body side and the elbow is lifted. This posture for holding gives a large burden on the surgeon and may cause the insertion of the insertion portion to be unstable.

Here, in the case of the endoscope in Literature 2, the insertion portion may be inserted into a nasal cavity with the front casing grasped and the first finger positioned on the finger rest/operation portion side. In this case, the surgeon has to twist the wrist in order to keep the insertion portion horizontal. This posture for holding also gives a large burden on the surgeon and may cause the bending operation of the bending portion and the insertion of the insertion portion to be unstable.

Japanese Patent Application Laid-Open Publication No. 2009-189684 indicates an endoscope in which an operation grasping portion is flexed, which is what is called "gun type". In this endoscope, an operation lever for performing operation to bend a bending portion is configured so as to rotate while facing a rear face of the operation portion. In the case of the endoscope having this configuration, a surgeon can easily keep the insertion portion horizontal and insert the insertion portion to a nasal cavity with the operation grasping portion stably grasped without the wrist being twisted and also can smoothly operate the operation lever with the thumb of the hand grasping the operation grasping portion.

However, surgeons wish to operate an operation lever not with a thumb, but, for example, a first finger for fine operation.

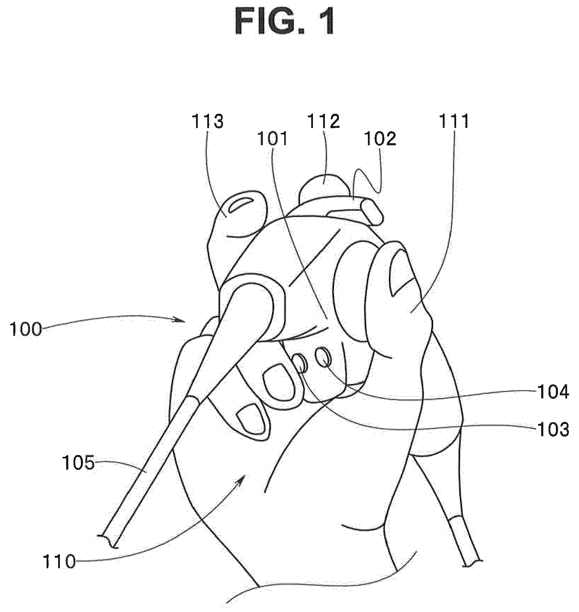

Also, as illustrated in Fig. 1, even with an endoscope 100 including a gun-type operation portion 101, a bending lever 102 can be operated with a first finger 112 of one hand 110 of a user by holding the operation portion 101 between a thumb 111 and a second finger 113.

Reference numerals 103 and 104 each denote a push-button switch. The first switch 103 is arranged, for example, as a release switch for shooting an endoscopic image to be displayed on a display apparatus (not-illustrated), and the second switch 104 is arranged as a freeze switch for temporarily freezing an endoscopic image displayed on the display apparatus. Reference numeral 105 denotes an insertion portion of the endoscope 100.

However, in an endoscope configured in such a manner that an operation lever is operated with the thumb of a hand holding an operation grasping portion such as indicated in Literature 3, if a surgeon grasps the operation grasping portion in such a manner as illustrated in Fig. 1 with priority given to operating the operation lever with the first finger, an insertion portion faces downward. Thus, the surgeon needs to twist the wrist in order to keep the insertion portion substantially horizontal, which gives a heavy burden on the surgeon. Also, the twisting of the wrist makes it difficult for the surgeon to operate the push-button switches provided at the operation portion, which may hinder endoscopic observation.

The present invention has been made in view of the aforementioned circumstances, and an object of the present invention is to provide an endoscope with high operability and high workability, the endoscope enabling a bending operation lever provided at an operation portion to be selectively operated with the thumb or another finger other than the thumb of a hand grasping the operation portion according to usage of the endoscope or the surgeon's preference.

### Disclosure of Invention

### Means for Solving the Problem

An endoscope according to an aspect of the present invention includes: an insertion portion including a bending portion that is bendable at least upward and downward on a distal end portion side; a cylindrical operation portion provided so as to be continuous with a proximal end side of the insertion portion, the operation portion having a first longitudinal axis substantially parallel to a longitudinal axis of the insertion portion and being capable of forming a part of a first grasping portion to be grasped by one hand of a user; a cylindrical extension portion provided so as to be continuous with a proximal end side of the operation portion, the extension portion having a second longitudinal axis extending in a downward bending direction of the bending portion and being capable of forming a part of the first grasping portion and a second grasping portion to be grasped by the one hand; and a bending lever including a first end and a second end, a first end portion pivotally provided at the operation portion, and a second end portion provided so as to extend from the first end portion in an upward bending direction of the bending portion, a finger of the one hand grasping the first grasping portion or the second grasping portion being placed on the second end portion when operation to bend the bending portion is performed.

### Brief Description of the Drawings

Fig. 1 is a diagram illustrating an example of a grasping state in an attempt to operate a bending lever provided at a conventional gun-type operation portion with a first finger;
Fig. 2 is a diagram for describing an endoscope according to the present invention, which is a view of one side of the endoscope;
Fig. 3 is a top view of the endoscope in Fig. 2 as viewed in the arrow Y3 direction;
Fig. 4 is a front view of the endoscope in Fig. 3 as viewed in the arrow Y4 direction;
Fig. 5 is a diagram illustrating a first grasping state of a grasping operation portion;
Fig. 6 is a diagram illustrating a second grasping state of the grasping operation portion;
Fig. 7 is a diagram illustrating a relationship between a shape of a hand and an extension portion in the second grasping state;
Fig. 8 is a diagram illustrating a modification of the second grasping state;
Fig. 9 is a diagram illustrating another configuration of the grasping operation portion; and
Fig. 10 includes diagrams each illustrating a grasping state of the grasping operation portion including a flexed extension portion in Fig. 9.

### Best Mode for Carrying Out the Invention

Embodiments of the present invention will be described below with reference to the drawings.

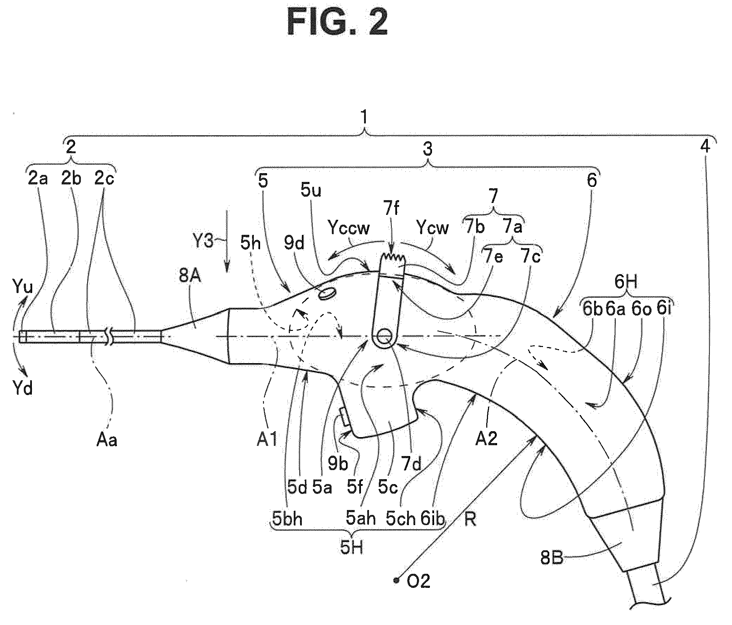

As illustrated in Fig. 2, an endoscope 1 mainly includes an insertion portion 2, a grasping operation portion 3 and a universal cable 4. The grasping operation portion 3 includes an operation portion 5 and an extension portion 6 integrally and fixedly provided. At the operation portion 5, a bending lever 7 is provided.

The insertion portion 2 is inserted to, for example, a nasal cavity.

In the insertion portion 2, a distal end portion 2a, a bending portion 2b and a flexible tube portion 2c are provided so as to be continuous in this order from the distal end side. Inside the distal end portion 2a, an image pickup apparatus (not-illustrated) including an image pickup device such as a CCD or C-MOS that picks up an image of a subject part is incorporated. Also, an image pickup apparatus including an image guide fiber instead of the image pickup device may be employed.

The bending portion 2b is configured so as to bend, for example, upward and downward. The bending portion 2b bends along with operation of the bending lever 7. The distal end portion 2a moves in an upward bending direction, which is indicated by arrow Yu in the figure, as a result of the bending portion 2b bending upward, and moves in a downward bending direction, which is indicated by arrow Yd, as a result of the bending portion 2b bending downward.

The flexible tube portion 2c has a flexibility that enables the flexible tube portion 2c to deform along a nasal cavity in a state in which the insertion portion 2 is inserted in the nasal cavity.

The operation portion 5 is provided so as to be continuous with the proximal end side of the insertion portion 2.

The bending lever 7 is provided on the extension portion 6 side of the operation portion 5, which is a proximal end portion of the operation portion 5.

As illustrated in Figs. 2 to 4, the bending lever 7 is provided with a lever body 7a and a finger rest 7b and has a substantially L-shape.

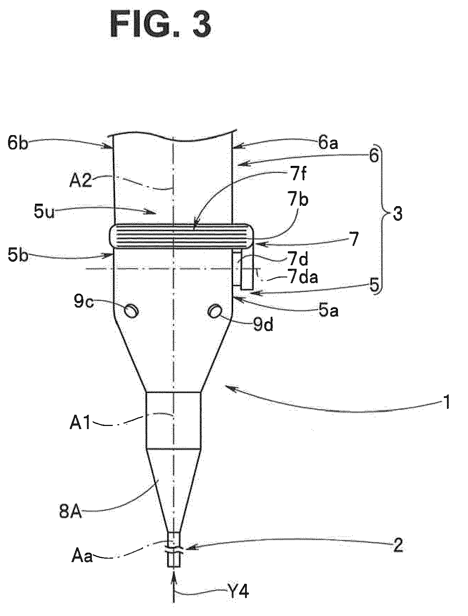

The lever body 7a includes a first end and a second end. The first end portion 7c is disposed on the one side face 5a side of the operation portion 5 so as to be pivotable via a pivot shaft member 7d. As illustrated in Fig. 3, a pivot axis 7da is substantially perpendicular to a first longitudinal axis A1.

The lever body 7a is provided so as to extend along the one side face 5a from the pivot shaft member 7d in the upward bending direction, and at the second end portion 7e, which is an end portion of the extension, a finger rest 7b is fixedly provided. The finger rest 7b includes a finger placing surface 7f. The finger placing surface 7f is provided with a slip stopper including, for example, recess and projection portions. The finger placing surface 7f of the finger rest 7b is provided a predetermined distance away from an upper face 5u of the operation portion 5.

The upper face 5u is a surface on the upward bending direction side of the bending portion 2b, and a surface opposite to the upper face 5u is a lower face 5d on the downward bending direction side.

The lever body 7a of the bending lever 7 configured as described above pivots about the pivot shaft member 7d as indicated in arrow Ycw and arrow Yccw along the upper face 5u of the operation portion 5. Therefore, a surface in the upper face 5u, the surface corresponding to a lever movement range, includes a curved surface.

The bending portion 2b is configured so as to bend, for example, upward upon the bending lever 7 being moved in the arrow Yccw direction, and bends downward, which is the opposite direction, upon the bending lever 7 being moved in the arrow Ycw direction.

Here, in the present embodiment, the lever body 7a is provided on the one side face 5a side of the operation portion 5. However, the lever body 7a may be provided on the other side face 5b side, which is a surface on the opposite side of the one side face 5a of the operation portion 5.

Also, the bending portion 2b may be configured so as to bend upward upon the bending lever 7 being moved in the arrow Ycw direction, and bend downward upon the bending lever 7 being moved in the arrow Yccw direction.

Furthermore, if the bending portion 2b is configured so as to bend upward, downward, rightward and leftward, a lever body for upward and downward bending or a lever body for rightward and leftward bending is provided at the one side face 5a of the operation portion 5, and a lever body that is different from the lever body provided at the one side face 5a is provided at the other side face 5b.

Also, the bending lever 7 may be configured in a rod-like joystick form. In this configuration, the rod portion of the joystick is provided so as to extend in the upward bending direction from the upper face 5u. A first end portion of the rod portion is pivotally provided inside the operation portion, and at a second end portion of the rod-like portion, for example, a spherical finger rest is provided.

Here, a configuration of each of the operation portion 5 and the extension portion 6 will be described with reference to Figs. 2 to 4.

First, the configuration of the operation portion 5 will be described.

The operation portion 5 illustrated in Figs. 2 to 4 is a hollow cylindrical member, and has a first longitudinal axis A1, which is an axis in a longitudinal direction parallel to a longitudinal axis Aa of the insertion portion 2. In the present embodiment, the longitudinal axis Aa and the first longitudinal axis A1 are coaxial to each other.

Note that a positional relationship between the longitudinal axis Aa and the first longitudinal axis A1 is not limited to the coaxial relationship and it is only necessary that the longitudinal axis Aa and the first longitudinal axis A1 be in substantially-parallel relationship.

The operation portion 5 includes a distal end face, the upper face 5u, the lower face 5d, a projection portion 5c, the one side face 5a and the other side face 5b, and a proximal end face. The distal end face is a surface perpendicular to the first longitudinal axis A1, and the insertion portion 2 extends from the distal end face. The upper face 5u is a surface that the finger rest 7b faces. The projection portion 5c projects from the lower face 5d. The one side face 5a is a surface at which the lever body 7a is disposed. The proximal end face is a surface to which a distal end face of the extension portion 6 is integrally fixed.

In the operation portion 5 in the present embodiment, an area 5h, which is indicated by the dashed line in Fig. 2, and a proximal end face 5ch of the projection portion 5c are configured so as to be capable of forming a part of a first grasping portion 5H to be grasped in a first grasping state. The area 5h is provided on each of the one side face 5a side and the other side face 5b side.

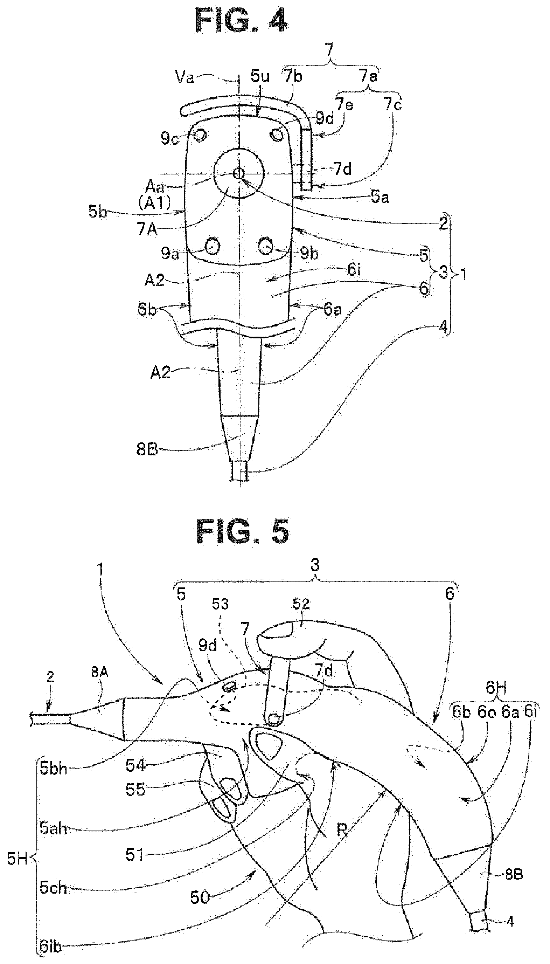

More specifically, the projection-portion proximal end face 5ch is an abutment surface. In the first grasping state illustrated in Fig. 5, for example, a base part between a thumb 51 and a first finger 52, or the palm of a right hand 50, which is one hand, are in abutment with the projection-portion proximal end face 5ch, whereby the position of the hand in the first grasping state is defined.

The area 5h in the one side face 5a is a first-grasping-portion first forming surface 5ah. In the first grasping state in Fig. 5, a ball part of the thumb 51 of the right hand is placed on the first-grasping-portion first forming surface 5ah. The area 5h in the other side face 5b is a first-grasping-portion second forming surface 5bh. In the first grasping state, a side portion on the first finger side of a second finger 53 of the right hand is placed on the first-grasping-portion second forming surface 5bh.

Here, when the surgeon grasps the operation portion 5 with his/her left hand, a side portion on the first finger side of the second finger of the left hand is placed on the first-grasping-portion first forming surface 5ah, a ball-side part of the thumb of the left hand is placed on the first-grasping-portion second forming surface 5bh, and thus the first-grasping-portion first forming surface 5ah and the first-grasping-portion second forming surface 5bh can form a part of the first grasping portion 5H to be grasped in the first grasping state.

In the present embodiment, push-button switches 9c and 9d are provided at respective predetermined positions in the upper face 5u, and push-button switches 9a and 9b are provided at respective predetermined positions in the projection portion distal end face 5f of the projection portion 5c.

The first switch 9a and the second switch 9b are arranged vertically symmetrically relative to a vertical axis Va in Fig. 4. Also, the third switch 9c and the fourth switch 9d are arranged also vertically symmetrically relative to the vertical axis Va. The switches 9c and 9d are arranged so as to be positioned in the upward bending direction relative to a position at which a proximal end portion of the insertion portion 2 is disposed.

In the present embodiment, the first switch 9a and the third switch 9c are provided as, for example, release switches for shooting an endoscopic image to be displayed on a display apparatus (not-illustrated). The second switch 9b and the fourth switch 9d are provided as, for example, freeze switches for temporarily freezing an endoscopic image displayed on the display apparatus.

Reference numeral 8A denotes a first bend preventing member. The first bend preventing member 8A covers an outer periphery of the flexible tube portion 2c joined to the operation portion 5 and prevents the flexible tube portion 2c from buckling.

Next, as a configuration of the extension portion 6 will be described.

The extension portion 6 illustrated in Figs. 2 to 4 is a hollow cylindrical member as with the operation portion 5. The extension portion 6 has a second longitudinal axis A2 that is different from the first longitudinal axis A1 in that the second longitudinal axis A2 is not parallel to the longitudinal axis Aa. In the present embodiment, the second longitudinal axis A2 is a circular arc-shape axis extending from the first longitudinal axis A1 in the downward bending direction of the bending portion 2b.

Thus, a proximal end face of the extension portion 6 is positioned in the downward bending direction of the bending portion 2b relative to the first longitudinal axis A1. The universal cable 4 extends from the proximal end face. As described above, the distal end face of the extension portion 6 is integrally fixed to the proximal end face of the operation portion 5.

Here, in Fig. 3, the first longitudinal axis A1 and the second longitudinal axis A2 are arranged in alignment with each other. However, the relevant configuration in the present invention is not limited to the configuration in which the first longitudinal axis A1 and the second longitudinal axis A2 are arranged in alignment with each other, and there may be a position gap between the first longitudinal axis A1 and the second longitudinal axis A2 while the first longitudinal axis A1 and the second longitudinal axis A2 are parallel to each other.

The extension portion 6 in the present embodiment includes the distal end face, an inner external surface 6i, an outer external surface 6o, one side face 6a, the other side face 6b and the proximal end face. The inner external surface 6i, which is a circular arc surface, is a surface forming a part on the center 02 side relative to the second longitudinal axis A2. The outer external surface 6o, which is a circular arc surface, is a surface forming a part on the outer side relative to the second longitudinal axis A2. The one side face 6a is a surface connecting the inner external surface 6i and the outer external surface 6o. The other side face 6b, which is a surface opposite to the one side face 6a, is a surface connecting the inner external surface 6i and the outer external surface 6o.

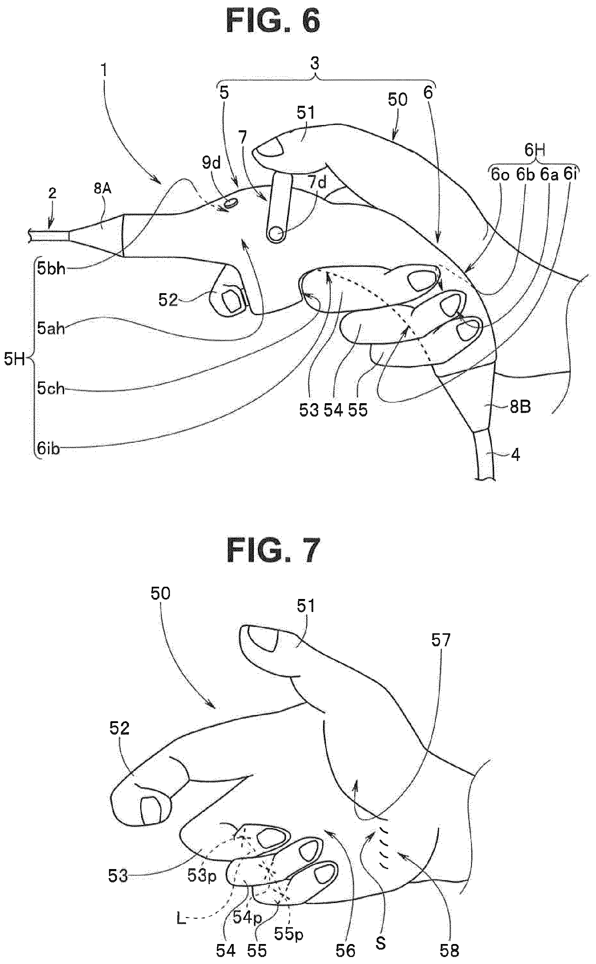

The extension portion 6 in the present embodiment is configured so as to be capable of forming a second grasping portion 6H to be grasped, for example, by a right hand 50 of one hand in a second grasping state as illustrated in Fig. 6, and forming a part of the first grasping portion 5H to be grasped in the first grasping state illustrated in Fig. 5.

As illustrated in Fig. 2, the second grasping portion 6H is formed by the one side face 6a, the other side face 6b, the inner external surface 6i and the outer external surface 6o. In the second grasping state illustrated in Fig. 6, the second grasping portion 6H is grasped by a thenar 57, a palm 56 and, for example, three fingers that are a fourth finger 55, a third finger 54 and a second finger 53 of a surgeon. Thus, a length in an axial direction of the second grasping portion 6H is set as a predetermined dimension in consideration of a width dimension of the palm 56.

In the second grasping state, the thenar 57 of the surgeon is placed on the outer external surface 6o, the palm 56 of the surgeon is placed on the other side face 6b, a ball part of a tip of the fourth finger 55, a ball part of a tip of the third finger 54 and a ball part of a tip of the second finger 53 of the surgeon are placed on the one side face 6a, a ball-side part between a first joint and a second joint of the fourth finger 55, a ball-side part between a first joint and a second joint of the third finger 54 and a ball-side part between a first joint and a second joint of the second finger 53 of the surgeon are placed on the inner external surface 6i.

In the present embodiment, an inner diameter R of the inner external surface 6i of the extension portion 6 is set to be no less than 60 mm and no more than 90 mm. The dimension of the inner diameter R ensures a stable second grasping state. The inner diameter R is a value obtained based on a line L connecting a point 53p on a part of the inner external surface 6i of the extension portion 6 corresponding to a part between the first joint and the second joint of the second finger 53, a point 54p on a part of the inner external surface 6i, the part corresponding to a part between the first joint and the second joint of the third finger 54, and a point 55p on a part of the inner external surface 6i, the part corresponding to a part between the first joint and the second joint of the fourth finger 55 in a state in which the palm of the hand is lightly clenched as illustrated in Fig. 7.

Here, an outer diameter of the outer external surface 6o is a value obtained based on a shell structure S formed by the thenar 57 and a hypothenar 58 in the state in which the palm of the hand is slightly clenched as illustrated in Fig. 7. The outer diameter of the outer external surface 6o is set to be no less than 100 mm and no more than 120 mm.

As described above, as a result of the inner diameter of the inner external surface 6i and the outer diameter of the outer external surface 6o, the inner external surface 6i and the outer external surface 6o being included in the extension portion 6, being set, an area of the palm of the hand, the area being in contact with the second grasping portion 6H, in the second grasping state increases, enabling provision of reliable and conformable grasping.

Here, if the surgeon grasps the extension portion 6 with his/her left hand, a thenar 57 may be placed on the outer external surface 6o, a palm 56 may be placed on the one side face 6a, a ball part of a tip of a fourth finger 55, a ball part of a tip of a third finger 54 and a ball part of a tip of a second finger 53 may be placed on the other side face 6b, a ball-side part between a first joint and a second joint of the fourth finger 55, a ball-side part between a first joint and a second joint of the third finger 54 and a ball-side part between a first joint and a second joint of the second finger 53 may be placed on the inner external surface 6i.

On the other hand, in the first grasping state illustrated in Fig. 5, a base part between the thumb 51 and the first finger 52 of the right hand of the surgeon is placed on the inner external surface 6i of the extension portion 6. In other words, an operation portion-side end face 6ib is a first-grasping-portion third forming surface. Therefore, the first grasping portion 5H is formed by the first-grasping-portion first forming surface 5ah, the first-grasping-portion second forming surface 5bh, the projection-portion proximal end face 5ch and the operation portion-side end face 6ib.

Here, reference numeral 8B denotes a second bend preventing member. The second bend preventing member 8B covers an outer periphery of the universal cable 4 connected to the extension portion 6 and prevents, for example, a signal cable inserted inside the universal cable 4, the signal cable being connected to an image pickup section, from being flexed sharply. Also, the second bend preventing member 8B may be used as a part of a grasping portion to be grasped by a surgeon with large hands.

Operation of the endoscope 1 configured as described above will be described.

The endoscope 1 according to the present embodiment is configured so that the grasping operation portion 3 can be grasped in the above-described first grasping state and can also be grasped in the second grasping state.

Therefore, if a surgeon wishes to operate the bending lever 7 with, for example, his/her first finger, as illustrated in Fig. 5, the grasping operation portion 3 of the endoscope 1 may be grasped in the first grasping state.

On the other hand, if the surgeon wishes to operate the bending lever 7 with, for example, his/her thumb, as illustrated in Fig. 6, the grasping operation portion 3 of the endoscope 1 may be grasped in the second grasping state.

When the first grasping state illustrated in Fig. 5 is selected, the surgeon grasps the first grasping portion 5H. In other words, with the palm part between the thumb 51 and the first finger 52 of the right hand 50 in abutment with the projection-portion proximal end face 5ch, the surgeon places the ball-side part of the thumb 51 on the first-grasping-portion first forming surface 5ah, and places the side portion on the first-finger side of the second finger 53 on the first-grasping-portion second forming surface 5bh. Then, the surgeon places the base part between the thumb 51 and the first finger 52 of the right hand on the operation portion-side end face 6ib and adjusts the position of the base part.

As a result, the surgeon can grasp the operation portion extension portion 3 with the right hand 50 in the first grasping state. In the first grasping state, the surgeon performs operation to pivot the bending lever 7 with a ball part of a tip of the first finger 52 on the finger placing surface 7f of the finger rest 7b. Here, the surgeon can perform fine lever operation by bending or extending the third joint, the second joint and the first joint of the first finger 52.

When the second grasping state illustrated in Fig. 6 is selected, the surgeon grasps the second grasping portion 6H. In other words, the surgeon grasps the second grasping portion 6H so as to grip the second grasping portion 6H lightly with the palm 56 and the fingers 53, 54 and 55 other than the first finger 52 of the right hand 50. Then, a ball portion between the first joint and the second joint of the second finger 53, a ball portion between the first joint and the second joint of the third finger 54 and a ball portion between the first joint and the second joint of the fourth finger 55 are placed in close contact on the inner external surface 6i included in the extension portion 6, and the thenar 57 and the hypothenar 58 are placed in close contact on the outer external surface 6o.

As a result, the surgeon can grasp the operation portion extension portion 3 with his/her right hand 50 in the second grasping state. In the second grasping state, the surgeon performs operation to pivot the bending lever 7 with the ball portion of the tip of the thumb 51 placed on the finger placing surface 7f of the finger rest 7b. Here, the surgeon can perform stable lever operation by bending or extending the second joint and the first joint of the thumb 51.

As described above, the grasping operation portion 3 is configured by integrally joining the operation portion 5 and the extension portion 6 to each other. Here, the proximal end face of the extension portion 6 is positioned in the downward bending direction of the bending portion 2b relative to the first longitudinal axis A1 of the operation portion 5, and the second longitudinal axis A2 of the extension portion 6 is set so as to have a circular arc shape.

As a result, the first grasping portion 5H that enables the bending lever 7 to be operated with the first finger and the second grasping portion 6H that enables the bending lever 7 to be operated by the thumb are provided in the grasping operation portion 3. The first grasping portion 5H is formed by the first-grasping-portion first forming surface 5ah, the first-grasping-portion second forming surface 5bh, the projection-portion proximal end face 5ch and the operation portion-side end face 6ib, and the second grasping portion 6H is formed by the one side face 6a, the other side face 6b, the inner external surface 6i and the outer external surface 6o.

Therefore, the surgeon can select a grasping state for grasping the grasping operation portion 3 according to usage of the endoscope 1 or the surgeon's preference and thereby operate the bending lever 7 with his/her thumb or first finger.

Also, the push-button third switch 9c and fourth switch 9d are provided vertically symmetrically relative to the vertical axis Va at the respective predetermined positions in the upper face 5u, and the push-button first switch 9a and second switch 9b are provided vertically symmetrically relative to the vertical axis Va at the respective predetermined positions in the projection portion distal end face 5f of the projection portion 5c. Then, a same function is assigned to the first switch 9a and the third switch 9c, and a function that is different from the function of the first switch 9a and the third switch 9c is provided to the second switch 9b and the fourth switch 9d or a function that is different from those of the others is provided to each of all of the switches from the first switch 9a to the fourth switch 9d. Consequently, irrespective of the first grasping state or the second grasping state, and also irrespective of the left hand or the right hand being used for grasping, lever operation and push-button operation of the switches can smoothly be performed.

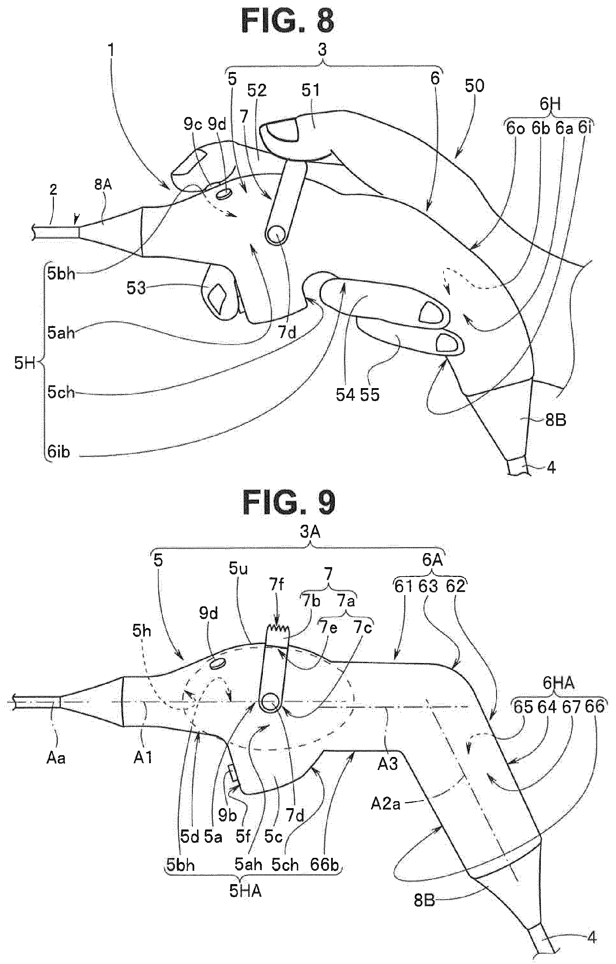

Note that, according to the endoscope including the grasping operation portion 3 configured as described above, a second grasping state can be provided by grasping the second grasping portion 6H of the extension portion 6 with a thenar 57, a palm 56, two fingers that are a third finger 54 and a fourth finger 55 as illustrated in Fig. 8. In this second grasping state, lever operation can be performed by a thumb 51 and push-button operation of the switches can be performed by a first finger 52 or a second finger 53. Also, in the above-described embodiment, in order to position the proximal end face of the extension portion 6 whose distal end face is integrally fixed to the proximal end face of the operation portion 5 in the downward bending direction of the bending portion 2b relative to the first longitudinal axis A1 of the operation portion 5, the second longitudinal axis A2 of the extension portion 6 is formed in a circular arc shape, enabling a grasping state for grasping the grasping operation portion 3 to be selected according to the usage of the endoscope 1 or the surgeon's preference, thus enabling bending lever 7 to be operated with the thumb or the first finger.

However, as illustrated in Fig. 9, operation and effects that are similar to those of the above-described embodiment may be obtained by providing a grasping operation portion 3A. Note that, in the below description, members that are the same as those of the above-described embodiment are provided with reference numerals that are the same as those of the embodiment and description thereof will be omitted.

A grasping operation portion 3A according to the present embodiment includes an operation portion 5 and an extension portion 6A. The extension portion 6A includes a first extension portion 61 and a second extension portion 62 integrally and fixedly provided.

Each of the first extension portion 61 and the second extension portion 62 is a hollow cylindrical member as with the operation portion 5. Each of a longitudinal axis of the first extension portion 61 and a longitudinal axis of the second extension portion 62 is a linear axis.

The first extension portion 61 has a third longitudinal axis A3, which is an axis in a longitudinal direction substantially parallel to a longitudinal axis Aa as with a first longitudinal axis A1. A length in the longitudinal direction of the first extension portion 61 is set to be no less than 20 mm and no more than 40 mm.

In the present embodiment, the longitudinal axis Aa and the third longitudinal axis A3 are arranged substantially in parallel to each other, and may have a position gap therebetween as illustrated in the figure or may be coaxial to each other as described above.

The second extension portion 62 has a second longitudinal axis A2a, which is inclined relative to the longitudinal axis A3. The second longitudinal axis A2a is a linear or circular arc axis. The second longitudinal axis A2a extends from the third longitudinal axis A3 side in the downward bending direction of the bending portion 2b. A proximal end face of the first extension portion 61 and a distal end face of the second extension portion 62 are smoothly connected with a flexed portion 63 provided therebetween. In other words, the extension portion 6A is configured as a flexed extension portion as a result of the inclusion of the first extension portion 61 and the second extension portion 62.

Therefore, a proximal end face of the second extension portion 62, which is a proximal end face of the extension portion 6A, is positioned in the downward bending direction of the bending portion 2b relative to the third longitudinal axis A3. A universal cable 4 extends from the proximal end face.

The extension portion 6A in the present embodiment is configured so as to be capable of forming a second grasping portion 6HA to be grasped in a second grasping state, and a part of a first grasping portion 5HA to be grasped in a first grasping state.

In the present embodiment, the second grasping portion 6HA includes a flexed-extension-portion other side face 65, a flexed-extension-portion outer face 66, a flexed-extension-portion inner surface 66, a flexed-extension-portion one side face 67 of the extension portion 6A.

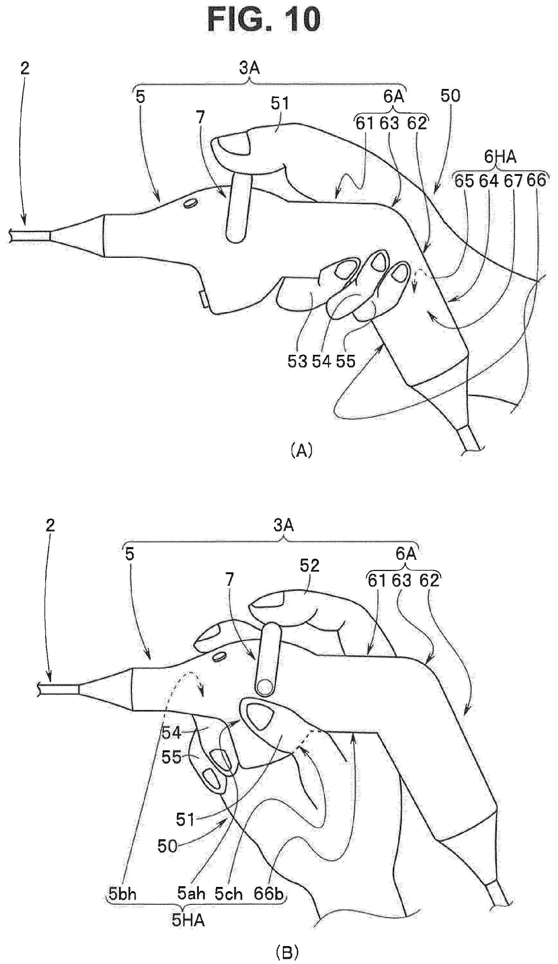

As illustrated in Fig. 10(A), in the second grasping state, a surgeon grasps the grasping operation portion 3A with a thenar 57, a palm 56 and, for example, three fingers that are a fourth finger 55, a third finger 54 and a second finger 53. In other words, in the second grasping state, the surgeon places the thenar 57 on the flexed-extension-portion outer face 64, places the palm 56 on the flexed-extension-portion other side face 65, places respective ball parts of the second finger 53, the third finger 54 and the fourth finger 55 on the flexed-extension-portion inner surface 66, and places respective tip portions of the second finger 53, the third finger 54 and the fourth finger 55 on the flexed-extension-portion one side face 67.

Here, if the surgeon grasps the extension portion 6A with his/her left hand, the surgeon places a thenar 57 on the flexed-extension-portion outer face 64, places a palm 56 on the flexed-extension-portion one side face 67, place respective ball portions of a second finger 53, a third finger 54 and a fourth finger 55 on the flexed-extension-portion inner surface 66, and places respective tip parts of the second finger 53, the third finger 54 and the fourth finger 55 on the flexed-extension-portion other side face 65.

On the other hand, in the present embodiment, the first grasping portion 5HA is formed by a first-grasping-portion first forming surface 5ah, a first-grasping-portion second forming surface 5bh and a projection-portion proximal end face 5ch of the operation portion 5 and an operation portion-side face 66b of the flexed-extension-portion inner surface 66 of the extension portion 6A.

As illustrated in Fig. 10(B), in the first grasping state, the surgeon place a base part between a thumb 51 and a first finger 52 of his/her right hand on the operation portion-side face 66b of the flexed-extension-portion inner surface 66. In other words, in the present embodiment, the operation portion-side face 66b of the flexed-extension-portion inner surface 66 is a first-grasping-portion third forming surface whose length in a longitudinal direction is set to be no less than 20 mm and no more than 40 mm.

In other words, in the first grasping state, with the base part between the thumb 51 and the first finger 52 placed on the operation portion-side face 66b, the surgeon places a ball-side part of the thumb 51 on the first-grasping-portion first forming surface 5ah, and places a side portion on the first finger side of the second finger 53 on the first-grasping-portion second forming surface 5bh.

Here, if the surgeon grasps the extension portion 6A with his/her left hand, with a base part between a thumb 51 and a first finger 52 placed on the operation portion-side face 66b, the surgeon places a ball-side part of the thumb 51 on the first-grasping-portion second forming surface 5bh, and places a side part on the first finger side of a second finger 53 on the first-grasping-portion first forming surface 5ah.

As described above, when the grasping operation portion 3A is formed by integrally joining the operation portion 5 and the extension portion 6A, the extension portion 6A is formed as a flexed extension portion. As a result, the first grasping portion 5HA that enables the bending lever 7 to be operated with a first finger and the second grasping portion 6HA that enables the bending lever 7 to be operated with a thumb can be provided in the grasping operation portion 3A.

Therefore, as in the above-described embodiment, the surgeon can select a grasping state for grasping the grasping operation portion 3A according to usage of the endoscope 1 or the surgeon's preference and operate the bending lever 7 with the thumb or the first finger.

The present invention is not limited only to the above-described embodiments and various modifications are possible without departing from the spirit of the invention.

The present application is filed claiming priority from Japanese Patent Application No. 2014-055376 filed in Japan on March 18, 2014, the disclosure of which is incorporated in the description, the claims and the drawings of the present application by reference.

## Claims

1. An endoscope comprising:
an insertion portion including, on a distal end portion side, a bending portion that is bendable at least upward and downward;
a cylindrical operation portion provided so as to be continuous with a proximal end side of the insertion portion, the operation portion having a first longitudinal axis substantially parallel to a longitudinal axis of the insertion portion and being capable of forming a part of a first grasping portion to be grasped by one hand of a user;
a cylindrical extension portion provided so as to be continuous with a proximal end side of the operation portion, the extension portion having a second longitudinal axis extending in a downward bending direction of the bending portion and being capable of forming a part of the first grasping portion and a second grasping portion to be grasped by the one hand; and
a bending lever including a first end and a second end, which has a first end portion pivotally provided at the operation portion, and a second end portion provided so as to extend from the first end portion in an upward bending direction of the bending portion, where a finger of the one hand grasping the first grasping portion or the second grasping portion is placed on the second end portion when operation to bend the bending portion is performed.

2. The endoscope according to claim 1, wherein the second longitudinal axis has a circular arc shape.

3. The endoscope according to claim 2, wherein an inner diameter of an inner external surface of the extension portion is no less than 60 mm and no more than 90 mm.

4. The endoscope according to claim 1, wherein the extension portion includes a first extension portion provided so as to be continuous with the proximal end side of the operation portion, the first extension portion having a third longitudinal axis substantially parallel to the longitudinal axis of the insertion portion and a second extension portion provided so as to be continuous with a proximal end side of the first extension portion, the second extension portion having a second longitudinal axis extending in the downward bending direction of the bending portion and being inclined relative to the third longitudinal axis.

5. The endoscope according to claim 4, wherein a length of the first extension portion is no less than 20 mm and no more than 40 mm.

6. The endoscope according to claim 4, wherein the first longitudinal axis and the third longitudinal axis are spaced a predetermined distance from each other.
